Europäisches Patentamt

European Patent Office

Office européen des brevets

(11) Publication number: **0 118 934**
**A1**

(12) **EUROPEAN PATENT APPLICATION**

(21) Application number: **84200158.8**

(22) Date of filing: **03.02.84**

(51) Int. Cl.³: **C 07 C 117/08**
**C 07 B 19/00**

(30) Priority: **14.02.83 NL 8300536**

(43) Date of publication of application:
**19.09.84 Bulletin 84/38**

(84) Designated Contracting States:
**BE CH DE FR GB IT LI NL**

(71) Applicant: **Océ-Andeno B.V.**
**Grubbenvorsterweg 8**
**NL-5928 NX Venlo(NL)**

(72) Inventor: **Bruggink, Alle**
**Maricollenweg 3**
**NL-5971 AS Grubbenvorst(NL)**

(72) Inventor: **Müris, Peter Gerardus**
**Moutzhofweg 144**
**NL-5926 RB Venlo(NL)**

(74) Representative: **Bleukx, Lucas Lodewijk Maria, Ir. et al,**
**Océ-Nederland B.V. Patents & Information Dept. Postbus**
**101**
**NL-5900 MA Venlo(NL)**

(54) **Optically active alpha-azido-p-hydroxyphenylacetic acid and its salts, and the preparation thereof.**

(57) Optically active α-azido-p-hydroxyphenylacetic acid and salts thereof. These novel optically active compounds can be prepared by reacting DL-α-azido-p-hydroxyphenylacetic acid with an optically active organic amine in a solvent and separating the crystallised salt from the solution. D(-)-α-azido-p-hydroxyphenylacetic acid is particularly suitable for the production of α-amino-p-hydroxybenzyl penicillins and cephalosporins.

EP 0 118 934 A1

Océ-Andeno B.V., at Venlo

Optically active  α-azido-p-hydroxyphenylacetic acid and its salts, and
the preparation thereof

This invention relates to optically active α-azido-p-hydroxyphenyl-
acetic acid and salts thereof, and more particularly D-(-)-α-azido-p-
hydroxyphenylacetic acid and salts thereof. The invention also relates
to a process for the preparation of these compounds.

The said compounds are novel and are particularly suitable as
starting product for the preparation of α-amino-p-hydroxybenzyl penicillins
and cephalosporins, more particularly for the preparation of 6-( α-amino-
p-hydroxybenzyl)-amino-6-methoxy penicillins. These products were
hitherto produced by means of D-(-)-α-amino-p-hydroxyphenylacetic acid.
The use of this known starting product, however, has the disadvantage
that the amino group, before the formation of the 6-methoxy penicillin,
must be protected very carefully and under critical conditions by means
of a group which after the penicillin preparation has to be removed under
equally dificult and very critical conditions. By-products may be formed
particularly during the removal of the protective group, so that the
yield of the known preparation process is unattractively low.
Direct coupling with the 6-methoxy penicillin fraction is possible when
using optically active α-azido-p-hydroxyphenylacetic acid, and after
this it is only necessary to convert theα-azido group into the required
amino function. This conversion can easily be carried out by means of a
catalytic reduction with hydrogen. Critical conditions, the use of
protective groups and by-product formation do not occur, so that high
yields are obtained.

α- Azido-p-hydroxyphenylacetic acid can also be used in the
preparation of the more conventional  α-amino-p-hydroxybenzyl penicillins
and cephalosporins. This process is of comparable value to the syntheses
hitherto used. However production via α-azido-p-hydroxyphenylacetic
acid is more attractive from the aspect of waste production. The
protective groups used in the known syntheses are ultimately  liberated
as waste products. The route via α-azido-p-hydroxyphenylacetic acid
results only in nitrogen as a waste product in the conversion to the
corresponding  α-amino compounds.

The novel optically active α-azido-p-hydroxyphenylacetic acids
can be prepared from the racemic form or directly from the raw materials of the racemic form. If is started from the racemic form, this can be done by dissolving DL-α-azido-p-hydroxyphenylacetic acid in a suitable solvent, such as an alcohol, ketone or ester whether or not mixed with water. One of the optical isomers can be crystallized out as a salt with the amine by the addition of an optically active organic base such as cinchonidine, ephedrine, cinchonine, brucine or morphine.

The diastereomeric salt of D-(-)-α-azido-p-hydroxyphenylacetic acid and cinchonidine or ephedrine can be crystallized out of a solution of the racemate with a surprisingly favourable yield. The corresponding salt of L-(+)-α-azido-p-hydroxyphenylacetic acid can be crystallized out with a very favourable yield by means of brucine. The optically active acids can be obtained in a conventional manner from the diastereomeric salts formed by conversion with a strong acid or a strong base which if required can be used in the form of an ion-exchanger.

The DL-α-azido-p-hydroxyphenylacetic acid used for the preparation of the novel optically active compounds can be obtained in a manner known per se for the preparation of azides by replacing the halogen atom in a corresponding halogen compound by an azido group. This reaction, however, has an unattractively low yield if used for the preparation of DL-α-azido-p-hydroxyphenylacetic acid. This azido compound can be obtained with a high yield by the reaction of α-hydroxy-p-hydroxy-phenylacetic acid with an azide, such as sodium azide, in an acid medium, e.g.aqueous sulphuric acid, hydrochloric acid or hydrobromic acid. It is notpossible to obtain α-azido-p-hydroxyphenylacetic acid in an alkalin medium,while the yield is low in a neutral medium. The best result is obtained in the presence of hydrochloric acid or hydrobromic acid. This process is additionally attractive because the starting material, which is also known as p-hydroxymandelic acid, is available on the market at a cheap price in large quantities or, if required, can easily be produced by condensation of phenol and glyoxylic acid. In the latter case it is also possible to dispense with the winning of p-hydroxymandeli acid. After the condensation of phenol with glyoxylic acid in an aqueous alkaline medium acidification can be carried out to the required degree of acidity and the reaction with sodium azide can be carried out.

An attractive process for the preparation of an optically active form of α-azido-p-hydroxyphenylacetic acid also comprises dispensing witl

winning the racemic form. To this end, after the reaction of p-
hydroxymandelic acid with sodium azide, the reaction mixture is extracted
with a water-immiscible solvent, such as an ester, and one of the
above-mentioned optically active amines is added to the extract. After
crystallization, the optically active form of α-azido-p-hydroxyphenyl-
acetic acid is obtained as a salt with the amine and is then processed
in known manner.

Example 1

Preparation of DL-α-azido-p-hydroxyphenylacetic acid with three
different methods

a) A mixture of 93 g (0.5 mol) of monohydrate of p-hydroxymandelic acid
and 39 g (0.6 mol) of sodium azide were stirred in 700 ml of water
at a temperature of 80°C for 3 hours. The reaction mixture was cooled
to 20°C and brought to a pH of 2.5 by means of concentrated hydro-
chloric acid. The DL-α-azido-p-hydroxyphenylacetic acid was
extracted with 2 x 300 ml of ethyl acetate and after concentration
of the extract was isolated via preparative column chromatography.
The support was silica gel and the eluant consisted of a mixture
of toluene, ethyl acetate and acetic acid in a ratio of 1:1:0.05.
The yield of DL-α-azido-p-hydroxyphenylacetic acid was 15%.

b) 30 ml of concentrated hydrochloric acid were added with continuous
stirring to a mixture of 18.6 g (0.1 mol) of monohydrate of p-
hydroxymandelic acid, 120 ml water and 13 g (0.2 mol) sodium azide.
The mixture was then stirred for an hour and a half at a reflux
temperature of 80°C and cooled to a temperature of 20°C The
aqueous phase of the reaction mixture was extracted with 200 ml
of tertiary butyl acetate and the extract was washed with 80 ml of
10% hydrochloric acid. 18.1 g (0.1 mol) of dicyclohexylamine were
then added, the mixture was stirred for 2 hours at a temperature
of 20°C and the resulting salt was filtered off. The weight after
drying was 27.4 g (yield 73%). The free acid was separated from the
salt by means of dilute hydrochloric acid. The precipitated
dicyclohexylamine hydrochloride was filtered off and the DL-α-azido-
p-hydroxyphenylacetatic acid was extracted from the filtrate with
tertiary butyl acetate and recovered by concentration. The yield
was 10 g.

c) A mixture of 22.5 g of phenol, 18.8 g (0.127 mol) of glyoxylic acid
(in the form of a 50% aqueous solution) and 50 ml of water was brought to a pH of 10.5 by means of a 50% caustic soda solution and stirred for 8 hours at 20°C. The reaction mixture was brought to a pH of 7 with hydrochloric acid and extracted with 2 x 25 ml of methyl isobutyl ketone. 16.3 g (0.25 mol) of sodium azide and 40 ml of concentrated hydrochloric acid were added to the aqueous phase and the mixture was then stirred at reflux temperature for two and a half hours and cooled to 20°C. The DL-$\alpha$-azido-p-hydroxyphenyl-acetic acid was recovered from the reaction mixture in the same way as described under 1b).

Example 2

Preparation of D-(-)-$\alpha$-azido-p-hydroxyphenylacetic acid

a) 10.9 g (0.037 mol) of cinchonidine were added with continuous stirring to a mixture of 7.72 g (0.04 mol) of DL-$\alpha$-azido-p-hydroxy-phenylacetic acid, 70 ml of isopropanol and 70 ml of tertiary butyl acetate. The mixture was stirred for 2 hours at 20°C and then the precipitated cinchonidine salt was filtered and dried. The yield was 7.9 g. The salt was dissolved in 45 ml of methanol at 60°C and after the addition of 50 ml of tertiary butyl acetate the mixture was cooled to 20°C and stirred for 2 hours at 20°C. The precipitated salt was filtered and dried. The yield was 4.4 g cinchonidine salt of D-(-)-$\alpha$-azido-p-hydroxyphenylacetic acid with a specific optical rotation $[\alpha]_D^{20}$= -190.8° (C=1 in 1 N hydrochloric acid).

4.3 g of the resulting salt were dissolved in 25 ml of water to which 4 ml of concentrated hydrochloric acid had been added. The free optically active acid was extracted with 2 x 20 ml of tertiary butylacetate and recovered by concentration after drying of the extract (with magnesium sulphate). The yield was 1.4 g of D-(-)-$\alpha$-azido-p-hydroxyphenylacetic acid having a melting point of 132 - 134°C and a specific optical rotation $[\alpha]_D^{20}$= -164° (C=1 in 96% ethanol).

b) 13.2 g (0.08 mol) of ephedrine were added with continuous stirring to a mixture of 17.4 g (0.09 mol) of DL-$\alpha$-azido-p-hydroxyphenylacetic acid, 250 ml tertiary butylacetate and 100 ml isopropanol. The mixture was stirred at 20°C for 6 hours and then the precipitated ephedrine salt was filtered off. The salt was dissolved in 34 ml of methanol at a temperature of 50°C. 100 ml of tertiary butyl acetate were added to the solution. The precipitate was filtered off and dried after 6 hours' stirring at 20°C. The yield was 5.5 g ephedrine salt of D-(-)-α-azido-p-hydroxyphenylacetic acid having a melting point of 148 - 149°C and a specific optical rotation $[\alpha]_D^{20}$ = -135° (C=1 in 1 N hydrochloric acid). 5.0 g of the resulting salt were dissolved in 60 ml of water and 15 ml of concentrated hydrochloric acid were added. The free optically active acid was extracted with 2 x 60 ml of tertiary butylacetate. After drying with magnesium sulphate and concentration of the extract, 2.15 g of D-(-)-α-azido-p-hydroxyphenylacetic acid were recovered.

Example 3

Preparation of L-(+)-α-azido-p-hydroxyphenylacetic acid
8.0 g of brucine were added with continuous stirring to a solution of 3.86 g (0.02 mol) of DL-α-azido-p-hydroxyphenylacetic acid in 60 ml of methanol. After 2 hours' stirring at 20°C the precipitated salt was filtered off and dried. The yield was 8.4 g. After recrystallization with 125 ml of methanol, 3.4 g of brucine salt of L-(+)-α-azido-p-hydroxyphenylacetic acid were recovered having a specific optical rotation $[\alpha]_D^{20}$ = + 58.6 (C=1 in 1 N hydrochloric acid). 3.3 g of this salt were dissolved in 20 ml of water to which 3 ml of concentrated hydrochloric acid had been added. The aqueous phase was extracted twice with 20 ml of tertiary butylacetate and 1 g of L-(+)-α-azido-p-hydroxyphenylacetic acid was obtained from the extract after drying (with magnesium sulphate) and concentration. This product had a melting point of 132 - 134°C and a specific optical rotation $[\alpha]_D^{20}$ = + 158° (C=1 in 96% ethanol).

0118934

## CLAIMS

1.      Optically active α-azido-p-hydroxyphenylacetic acid and salts thereof.

2.      D-(-)-α-azido-p-hydroxyphenylacetic acid and salts thereof.

3.      A process for the preparation of a compound according to claim 1, characterised in that DL-α-azido-p-hydroxyphenylacetic acid is reacted in a solvent with an optically active organic amine, this diastereomeric salt formed is crystallized out and the free acid is split off if necessary.

4.      A process according to claim 3, characterised in that the optically active organic amine used is cinchonidine, ephedrine or brucine.

5.      Salt of D-(-)-α-azido-p-hydroxyphenylacetic acid with cinchonidine.

6.      Salt of D-(-)-α-azido-p-hydroxyphenylacetic acid with ephedrine.

7.      Salt of L-(+)-α-azido-p-hydroxyphenylacetic acid with brucine.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | CH-A- 420 105 (BEECHAM) <br> * Claims * | 1,3,4 | C 07 C 117/08 <br> C 07 B 19/00 |
| Y | FR-A-2 060 460 (A. COLL) <br> * Claims * | 1,3,4 | |
| Y | DE-A-1 948 667 (ASTRA) <br> * Claims * | 1,3,4 | |
| Y | DE-A-2 125 979 (ASTRA) <br> * Claims * | 1,3,4 | |
| Y | CHEMICAL ABSTRACTS, vol. 94, no. 17, 27th April 1981, page 727, no. 139431a, Columbus, Ohio, US & JP - A - 80 94 350 (SAGAMI CHEMICAL RESEARCH CENTER) 17-07-1980 * Abstract * | 1 | |

TECHNICAL FIELDS SEARCHED (Int. Cl. ³)

C 07 C 117/00
C 07 B 19/00

The present search report has been drawn up for all claims

| Place of search | Date of completion of the search | Examiner |
|---|---|---|
| THE HAGUE | 22-05-1984 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82